# EUROPÄISCHE PATENTSCHRIFT

(11) **EP 0 733 221 B1**
(45) Veröffentlichungstag und Bekanntmachung des Hinweises auf die Patenterteilung: **08.01.2003**
(21) Anmeldenummer: 95932637.2
(22) Anmeldetag: 28.09.1995
(51) Int. Cl.: G02B 1/04, C08L 79/04, C08G 73/06

(54) **OPTISCHE ELEMENTE DER NACHRICHTENTECHNIK AUS KUNSTSTOFF**
PLASTIC OPTICAL COMPONENTS FOR COMMUNICATION ENGINEERING
ELEMENTS OPTIQUES EN PLASTIQUE POUR LA TECHNIQUE DES COMMUNICATIONS

(30) Priorität: 08.10.1994 DE 4435992
(43) Veröffentlichungstag der Anmeldung: 25.09.1996
(73) Patentinhaber: FRAUNHOFER-GESELLSCHAFT ZUR FÖRDERUNG DER ANGEWANDTEN FORSCHUNG E.V., 80636 München (DE)
(72) Erfinder: BAUER, Monika, D-12619 Berlin (DE); KRÜGER, Harmut, D-12489 Berlin (DE); BRÄUER, Andreas, D-07646 Rabis (DE)
(74) Vertreter: Olgemöller, Luitgard, Dr.
(86) Internationale Anmeldenummer: DE9501353
(87) Internationale Veröffentlichungsnummer: WO96011415

(56) Entgegenhaltungen:
- US-A- 3 839 442
- US-A- 4 334 045
- US-A- 4 414 366
- US-A- 4 902 752

## Beschreibung

Die Erfindung betrifft optische Elemente aus Kunststoff und insbesondere solche aus Polycyanuratharzen.

Polymere sind zunehmend interessante Materialien für die Optik, Mikrooptik, integrierte Optik und Mikrosystemtechnik. Sie finden dabei Verwendung in optischen Geräteteilen sowie in speziellen Optiken als Linsen, Prismen, zur Fixierung optischer Systeme, als Trägermaterial für optische Schichten sowie als transparentes Beschichtungsmaterial für Spiegel und Linsen.

So schlagen die US-Patentschriften 4,334,045 und 4,414,336 die Verwendung von vernetzten Polycyanurat-Polymeren, ggf. in Mischung mit thermoplastischen Polymeren, für die Herstellung von Windschutzscheiben, Verkleidungen oder Türverglasungen vor, da man bei entsprechend umsichtiger Verfahrensführung Polymere hierfür mit nur sehr leichter Gelbfärbung erhalten könne.

Polymere können auch für optische Fasern und zur Erzeugung von Wellenleiterstrukturen eingesetzt werden. Ihr allgemeiner Vorteil liegt in der günstigen technologischen Verarbeitbarkeit und ihrer im Vergleich zu Glas geringeren Dichte.

Insbesondere die Anwendung als Wellenleiter stellt mannigfaltige Anforderungen an das Polymer. Die Brechzahl des Materials soll möglichst variierbar und an bestimmte Substrate anpaßbar sein. Bei einer Anwendung in der optischen Nachrichtentechnik werden geringe Materialabsorptionen bei 1,3 und 1,55 µm gefordert. Die Dämpfungsverluste durch Volumendefekte (Inhomogenitäten, Mikroblasen) sind zu minimieren. Neben bestimmten technologischen Anforderungen, wie Schichtherstellung und Strukturierbarkeit sind insbesondere die thermische und thermo-mechanische Stabilität, angepaßte Ausdehnungskoeffizienten und ein geringer Schrumpf Voraussetzungen für einen Einsatz von Polymeren für Wellenleiterstrukturen in der integrierten Optik.

Bisher für optische Anwendungen genutzte Kunststoffe sind Polymethacrylate und Polycarbonate. Ihr Brechzahlbereich ist jedoch mit 1,49 bzw. 1,58 relativ eingeschränkt und nicht ohne weiteres variierbar. Beide Polymerklassen weisen eine excellente optische Transparenz auf, sind jedoch bedingt durch ihre chemische Struktur thermisch und thenno-mechanisch nicht besonders stabil. So ist Polycarbonat beispielsweise durch seinen relativ niedrigen Glasumwandlungsbereich bei Temperaturen über 130 °C praktisch nicht mehr einsetzbar.

Andere Hochleistungspolymere weisen Glasumwandlungsbereiche (T_{g}) von > 180 °C auf. Beispiele hierfür sind Polyarylethersulfone, Polyarylsulfone, Polyaryletherketone, Polyimide und Polyetherimide, die sich, verglichen mit Polymethacrylat und Polycarbonat, jedoch meist schwieriger verarbeiten lassen. Die Anwendung dieser Hoch-T_{g}-Polymere für optische Systeme wird in verschiedenen Patentschriften beschrieben, so in JP-A- 61-144738, JP-A- 61-005986, DE-A-39 15 734, US-A-4 477 555, EP-A-0 254 275, DE-A-34 29 074, DE-A-39 27 498, DE-A-42 28 853, DE-A-36 36 399. Ein weiterer Nachteil dieser Systeme ist die vergleichsweise hohe optische Dämpfung bei den nachrichtentechnisch relevanten Wellenlängen von 1,3 und 1/55 µm.

Der Erfindung lag demzufolge die Aufgabe zugrunde, leicht verarbeitbare, brechzahlvariable, thermisch und thermo-mechanisch stabile Polymere mit einer geringen Absorption bei 1,3 und 1,55 um, die für die Herstellung von optischen Elementen der Nachrichtentechnik geeignet sind, sowie daraus hergestellte optische Elemente der Nachrichtentechnik bereitzustellen.

Diese Aufgabe wird mit einem optischen Element der Nachrichtentechnik der eingangs bezeichneten Art gelöst, das aus einem Polycyanuratharz hergestellt ist.

Es wurde überraschend gefunden, daß Polycyanuratharze zur Herstellung optischer Elemente mit den oben genannten erwünschten Eigenschaften besonders gut geeignet sind. Dabei handelt es sich überwiegend um bekannte Produkte aus herkömmlichen Polycyanat-Ausgangsmaterialien, wie sie in der Kunststoffindustrie vielfach eingesetzt werden. Entsprechend sind die Ausgangsmaterialien, Herstellungsverfahren und Verarbeitungsverfahren dieser Polycyanuratkunststoffe bekannt.

Besonders geeignet für die erfindungsgemäßen optischen Elemente sind Polycyanuratharze, die aus den nachstehend aufgeführten Verbindungen erhalten werden. 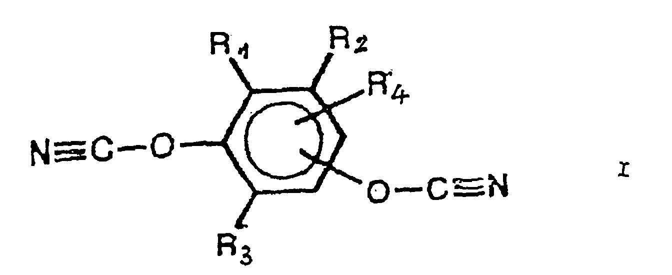 worin R¹ bis R⁴ unabhängig voneinander Wasserstoff, C₁-C₁₀-Alkyl, C₃-C₈-Cycloalkyl, C₁-C₁₀-Alkoxy, Halogen oder Phenyl ist, wobei die Alkyl- oder Arylgruppen fluoriert oder teilfluoriert sein können, 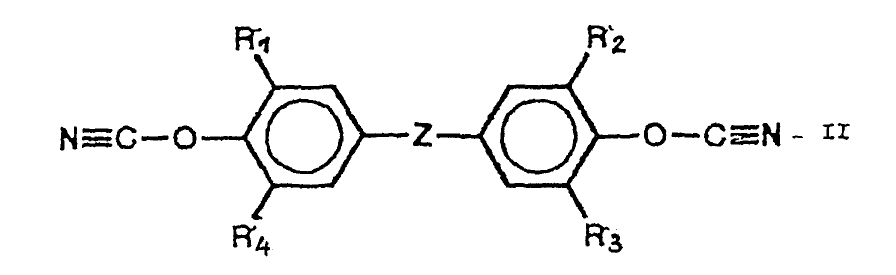 worin Z eine chemische Bindung, SO₂, CF₂, CH₂, CH(CH₃), Isopropyl, Hexafluoroisoporpyl, C₁-C₁₀-Alkyl, O, NR⁵, N=N, CH=CH, COO, CH=N, CH=N-N=CH, Alkyloxyalkyl mit C₁-C₈-Alkyl, S oder 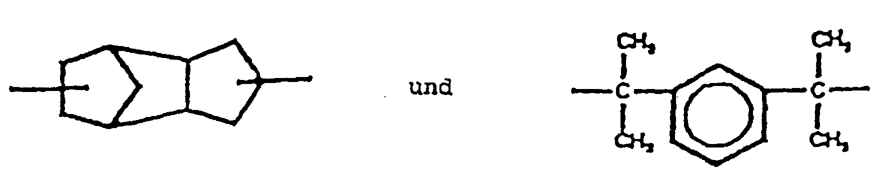 ist, 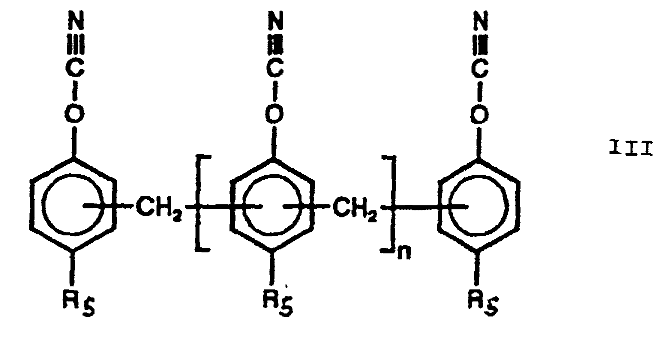 worin R⁵ Wasserstoff oder C₁-C₁₀-Alkyl ist und n 0 bis 20 ist sowie
Dicyanaten eines perhalogenierten Dihydroxyalkans, insbesondere mit bis zu 10 Kohlenstoffatomen, IV
sowie Mischungen der Polycyanate der Formeln I bis IV.

Die Eigenschaften der vorstehend genannten Polycyanuratharze können dadurch vorteilhaft beeinflußt werden, daß sie zum einen in Mischungen eingesetzt werden, zum anderen aber mit Phenolen, z. B. der Struktur Va bis c, worin R Wasserstoff ist und R¹ bis R⁵ wie vorstehend definiert sind, 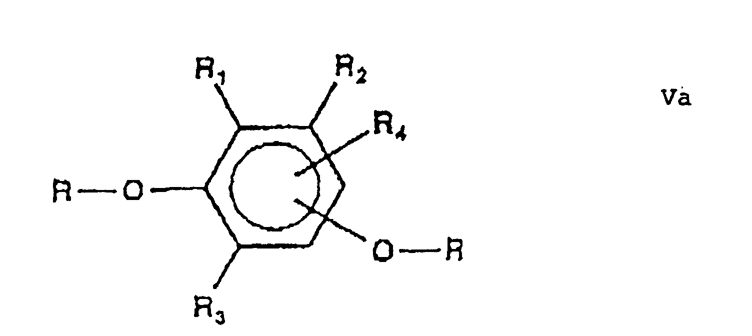 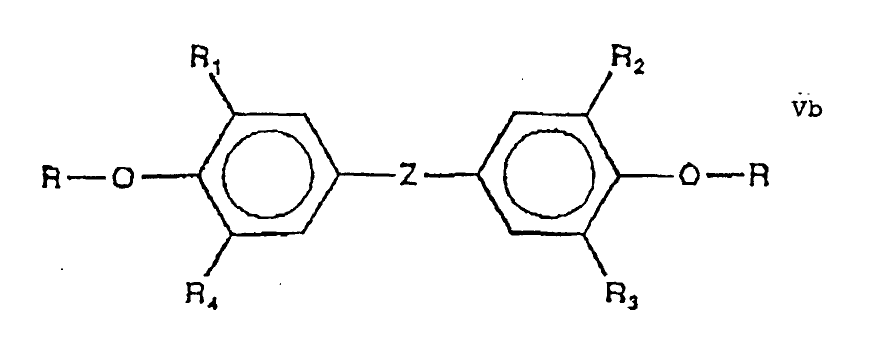 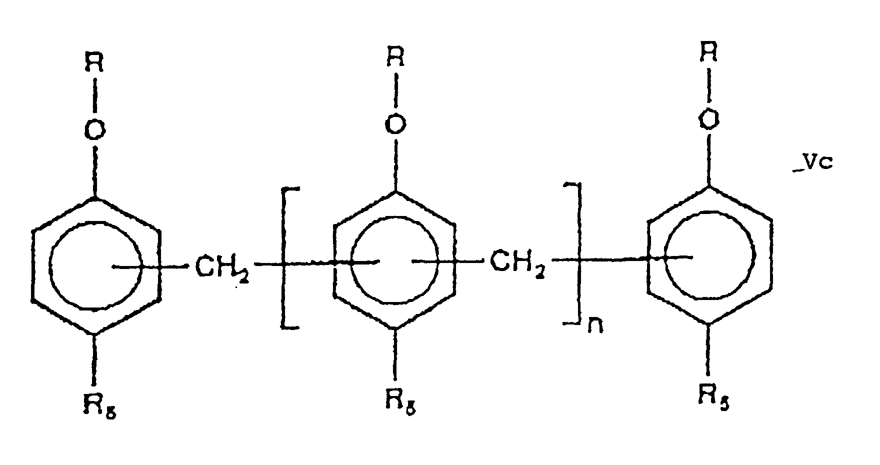 aromatischen Glycidethern der Struktur Va bis c, worin R Glycidyl ist, oder Glycidylanilinen, z. B. der Grundstruktur VIa bis c 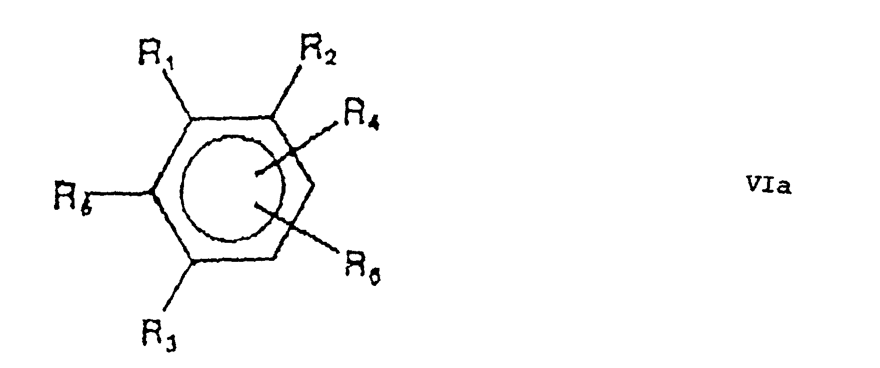 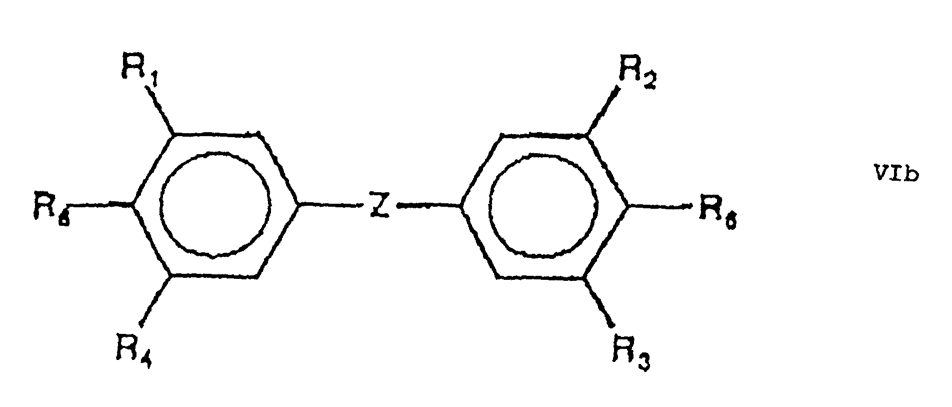 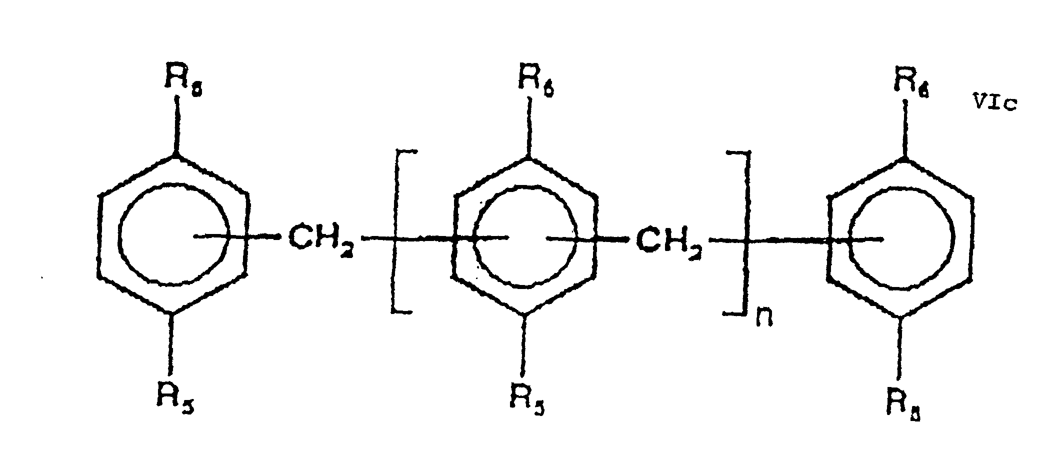 worin R₆ N(R₇)₂ mit R₇ Glycidyl ist, unter vollständiger Aushärtung umgesetzt werden.

Derartige Coreaktionsprodukte mit Phenolen, aromatischen Glycidethern und Glycidylanilinen sind ebenfalls grundsätzlich bekannt. Beispielsweise können Polycyanurätharze erhalten werden, die, bezogen auf das verwendete Polycyanat oder Polycyanatgemisch, 1 bis 60 Mol% Phenol, 1 bis 99 Mol.% Glycidether oder 1 bis 99 Mol% Glycidylanilin oder Gemische dieser Komponenten enthalten.

Die erfindungsgemäß verwandten Polycyanuratharze haben insbesondere eine Glasübergangstemperatur T_{g} von 100 bis 250 °C, besonders bevorzugt von 180 bis 250 °C. Ihr Brechungsindex bei 633 nm liegt vorzugsweise im Bereich von 1,45 bis 1,70, besonders bevorzugt im Bereich von 1,55 bis 1,65.

Erfindungsgemäß können optische Dämpfungen im Bereich von 0,1 bis 1,0 dB/cm bei 1,3 bzw 1,55 µm erreicht werden.

Geeignet sind die Polycyanuratharze zur Herstellung von Wellenleiterstrukturen, auf dem Gebiet der Nachrichtentechnik einzusetzenden Linsen, Prismen, korrigierten Linsensystemen, optischen Lichtleitfasern und Trägern für optische Schichten sowie zum Verkleben optischer Komponenten der Nachrichtentechnik und zur Faserankopplung, wie auch für zahlreiche andere Zwecke auf dem Gebiet der Nachrichtentechnik.

Die verwendeten Polycyanuratharze können erfindungsgemäß dadurch erhalten werden, daß Gemische verschiedener Dicyanate in der Polymerbildungsreaktion eingesetzt werden. Weiterhin können die Polycyanurate dadurch erhalten werden, daß Dicyanate in Coreaktionen mit Phenolen, aromatischen Glycidethern oder Glycidylanilinen zu den erfindungsgemäßen Produkten führen. Die Brechzahl der beschriebenen Polycyanuratharze ist durch die Mischung der Monomerkomponenten in einem weiten Bereich (1,45 bis 1,70) variierbar und einstellbar. Die thermische Stabilität der Polymere liegt bei bis zu 250 °C. Die Glasumwandlungsbereiche der erfindungsgemäßen Harze liegen zwischen 100 und 250 °C, und insbesondere bei > 180 °C.

Die beschriebenen Harze weisen im Vergleich zu anderen Hoch-Tg-Polymeren, z. b. Polyimiden, sehr niedrige optische Verluste bei 1,3 und 1,55 µm auf. Die leichte Verarbeitbarkeit der erfindungsgemäßen Materialien ist dadurch gegeben, daß sie bereits in einer löslichen Prepolymerstufe aus der Lösung mittels spin-coating oder aus der Schmelze mittels Präge- oder Abformtechniken verarbeitet werden können. Die Polymerisate weisen auf verschiedenen Substraten eine gute Haftung auf. Die Endverarbeitung in der Schicht erfolgt durch thermische Härtung.

Gegenstand der Erfindung ist ferner die Verwendung von Polycyanuratharzen, insbesondere solchen, wie sie vorstehend näher definiert sind, zu Herstellung von optischen Elementen.

Die Erfindung wird durch die nachfolgenden Beispiele näher erläutert.

### Beispiel 1

100 g Dicyanat des Bisphenol A (Verbindung II mit R₁ bis R₄ = H, Z = Isopropyl) werden auf 180 °C unter Rühren und Inertgasatmosphäre erwärmt. Nach 350 min wird das entstandene Prepolymer abgekühlt. Es erstarrt als transparente, amorphe, leicht gelblich gefärbte Masse. Mittels IR-Spektroskopie wird ein OCN-Umsatz von 41 % bestimmt.

Das Prepolymer ist in den gebräuchlichen organischen Lösemitteln löslich. Aus einer 40%igen Lösung in Cyclohexanon werden duch Spin-coating Filme im Dickenbereich von 1 bis 10 µm erhalten. Nach Tempern und Aushärten der Schicht bei zuletzt 200 °C wird bei 633 nm eine Brechzahl von 1,6095 erhalten. Die optische Dämpfung bei 1,32 µm betrug 0,39 dB/cm, bei 1,55 µm 1,1 dB/cm.

### Beispiel 2

10 g Dicyanat eines substituierten Bisphenol A (Verbindung II mit R₁ - R₄ = H, Z = Hexafluoroisopropyl) werden bei 180 °C für 70 h bis zur völligen Aushärtung erwärmt. Es wird ein transparenter, amopher, farbloser Körper erhalten. Mittels IR-Spektroskopie wird der OCN-Umsatz nachgewiesen.

Der entstandene Formkörper kann mechanisch bearbeitet werden. Durch Polieren werden Stirnflächen zum Einkoppeln des Laserstrahls erhalten. Es wird bei 663 nm eine Brechzahl von 1,543 bestimmt. Die optische Dämpfung bei 1,32 µm betrug 0,6 dB/cm, bei 1,55 µm 0,7dB/cm.

### Beispiel 3

25 g Dicyanat eines substituierten Bisphenol A (Verbindung II mit R₁ - R₄ = CH₃, Z = CH₂) werden bei 180 °C für 80 h bis zur völligen Aushärtung erwärmt. Es wird ein transparenter, amorpher, gelblicher Körper erhalten. Mittels IR-Spektroskopie wird der OCN-Umsatz nachgewiesen.

Der entstandene Formkörper kann mechanisch bearbeitet werden. Durch Polieren werden Stirnflächen zum Einkoppeln des Laserstrahls erhalten. Es wird bei 633 nm eine Brechzahl von 1,579 (bei 1,3 µm 1,558) bestimmt. Die optische Dämpfung bei 1,32 µm betrug 0,6 dB/cm, bei 1,55 µm 0,6 dB/cm.

### Beispiel 4

7 g Dicyanat eines substituierten Bisphenol A (Verbindung II mit R₁ - R₄ = CH₃, Z = CH₂) und 3 g Dicyanat des Bisphenol A (Verbindung II mit R₁ - R₄ = H, Z = Isopropyl) werden bei 120 °C für 92 h unter Zugabe von 2 % eines Katalysators, bestehend aus 200 Teilen Phenol und 1 Teil Cu(acac)₂, bis zur völligen Aushärtung erwärmt. Es wird ein transparenter, amorpher, gelblicher Körper erhalten. Mittels IR-Spektroskopie wird der OCN-Umsatz nachgewiesen.

Der entstandene Formkörper kann mechanisch bearbeitet werden. Durch Polieren werden Stirnflächen zum Einkoppeln des Laserstrahls erhalten. Es wird bei 633 nm eine Brechzahl von 1,592 bestimmt.

### Beispiel 5

3 g Dicyanat eines substituierten Bisphenol A (Verbindung II mit R₁ - R₄ = H, Z = Hexafluoroisopropyl) und 7 g Dicyanat des Bisphenol A (Verbindung II mit R₁ - R₄ = H, Z = Isopropyl) werden bei 120 °C für 92 h unter Zugabe von 2 % eines Katalysators, bestehend aus 200 Teilen Phenol und 1 Teil Cu(acac)₂, bis zur völligen Aushärtung erwärmt. Es wird ein transparenter, amorpher, schach gelblicher Körper erhalten. Mittels IR-Spektroskopie wird der OCN-Umsatz nachgewiesen.

Der entstandene Fromkörper kann mechanisch bearbeitet werden. Durch Polieren werden Stirnflächen zum Einkoppeln des Laserstrahls erhalten. Es wird bei 633 nm eine Brechzahl von 1,592 bestimmt.

## Patentansprüche

1. Optische Elemente der Nachrichtentechnik aus Kunststoff, **dadurch gekennzeichnet, daß** der Kunststoff ein Polycyanuratharz ist.

2. Optisches Element nach Anspruch 1, **dadurch gekennzeichnet, daß** das Polycyanuratharz ausgehend von wenigstens einem Polycyanat der nachstehenden Formeln I bis IV erhältlich ist 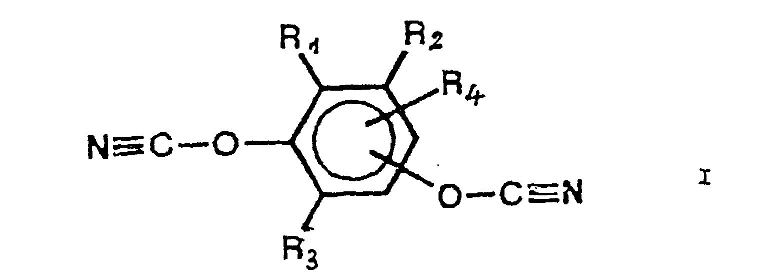 worin R¹ bis R⁴ unabhängig voneinander Wasserstoff, C₁-C₁₀-Alkyl, C₃-C₈-Cycloalkyl, C₁-C₁₀-Alkoxy, Halogen oder Phenyl ist, wobei die Alkyl- oder Arylgruppen fluoriert oder teilfluoriert sein können, 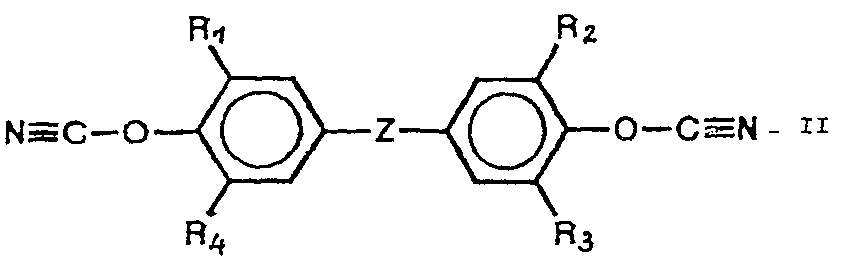 worin Z eine chemische Bindung, SO₂, CF₂, CH₂, CH(CH₃), Isopropyl, Hexafluoroisoporpyl, C₁-C₁₀-Alkyl, O, NR⁵, N=N, CH=CH, COO, CH=N, CH=N-N=CH, Alkyloxyalkyl mit C₁-C₈-Alkyl, S oder 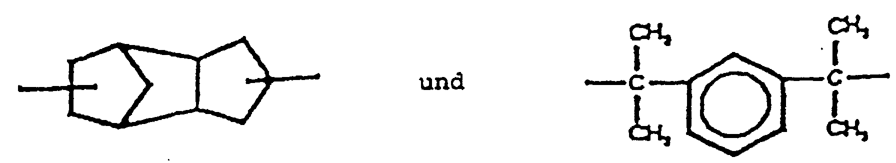 ist, 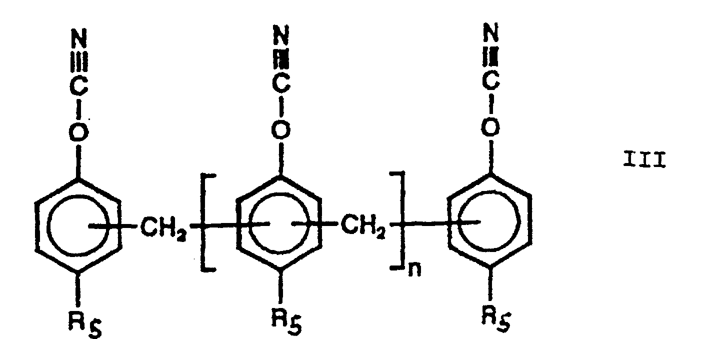 worin R⁵ Wasserstoff oder C₁-C₁₀-Alkyl ist und n 0 bis 20 ist sowie
einem Dicyanat eines perhalogenierten Dihydroxyalkans IV, insbesondere mit bis zu 10 Kohlenstoffatomen,
sowie Mischungen der Polycyanate I bis IV.

3. Optisches Element nach Anspruch 1 oder 2, **dadurch gekennzeichnet, daß** das Polycyanuratharz von einem Bisphenol-A-Dicyanat abgeleitet ist.

4. Optische Elemente nach einem der Ansprüche 1 bis 3, **dadurch gekennzeichnet, daß** die Polycyanuratharze Coreaktionsprodukte der Polycyanate mit Phenolen, aromatischen Glycidethern oder Glycidylanilinen sind.

5. Optische Elemente nach Anspruch 4, **dadurch gekennzeichnet, daß** die Polycyanuratharze, bezogen auf eingesetztes Polycyanat, 1 bis 60 Mol% Phenol, 1 bis 99 Mol% Glycidyether oder 1 bis 99 Glycidylanilin oder Gemische dieser Komponenten enthalten.

6. Optische Elemente nach einem der vorstehenden Ansprüche, **dadurch gekennzeichnet, daß** das Polycyanuratharz eine Glasübergangstemperatur von 100 bis 250 °C, vorzugsweise 180 bis 250 °C, aufweist.

7. Optische Elemente nach einem der vorstehenden Ansprüche, **gekennzeichnet durch** einen Brechungsindex bei 633 nm im Bereich von 1,45 bis 1,70, vorzugsweise 1,55 bis 1,65.

8. Optische Elemente nach einem der vorstehenden Ansprüche, **gekennzeichnet durch** eine optische Dämpfung bei 1,3 bzw. 1,55 µm im Bereich von 0,1 bis 1,0 dB/cm.

9. Optische Elemente nach einem der vorstehenden Ansprüche, **dadurch gekennzeichnet, daß** sie durch Spin-coating des gelösten Polycyanuratharz erhalten wurden.

10. Optische Elemente nach einem der Ansprüche 1 bis 8, **dadurch gekennzeichnet, daß** sie durch Abform- bzw. Prägetechniken aus der Schmelze erhalten wurden.

11. Optische Elemente nach einem der Ansprüche 1 bis 10, **dadurch gekennzeichnet, daß** das Polycyanuratharz thermisch, ggf. in Gegenwart eines Katalysators, gehärtet wurde.

12. Wellenleiterstrukturen, auf dem Gebiet der Nachrichtentechnik einzusetzende Linsen, Prismen, korrigierte Linsensysteme, optische Lichtleitfasern und Träger für optische Schichten sowie Klebstoffe optischer Komponenten der Nachrichtentechnik aus einem Polycyanuratharz, wie in einem der Ansprüche 1 bis 11 definiert.

13. Verwendung von Polycyanuratharzen, wie in einem der Ansprüche 1 bis 11 definiert, zur Herstellung optischer Elemente der Nachrichtentechnik, insbesondere solcher nach Anspruch 11.

## Claims

1. Optical elements of information technology made from plastic, **characterised in that** the plastic is a polycyanurate resin.

2. Optical element according to claim 1, **characterised in that** the polycyanurate resin can be obtained starting from at least one polycyanate of the formulae I to IV below 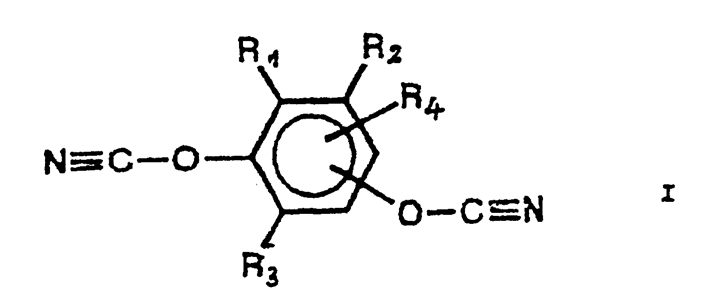 wherein R¹ to R⁴ independently of one another is hydrogen, C₁-C₁₀ alkyl, C₃-C₈ cycloalkyl, C₁-C₁₀ alkoxy, halogen or phenyl, wherein the alkyl or aryl groups may be fluorinated or partly fluorinated, 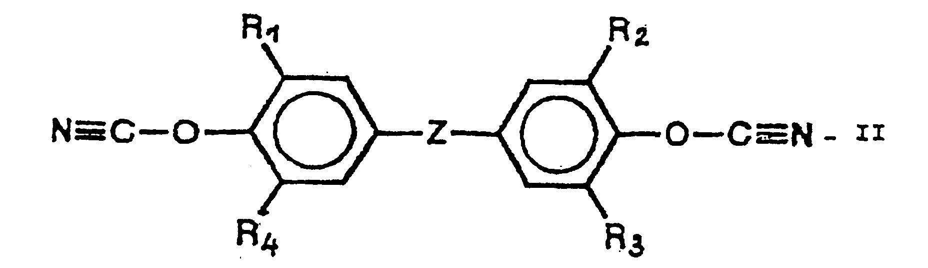 wherein Z is a chemical bond, SO₂, CF₂, CH₂, CH(CH₃), isopropyl, hexafluoroisopropyl, C₁-C₁₀ alkyl, O, NR⁵, N=N, CH=CH, COO, CH=N, CH=-N-N=CH, alkyloxyalkyl having C₁-C₈ alkyl, S or 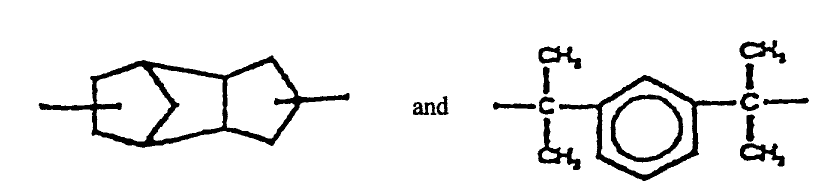 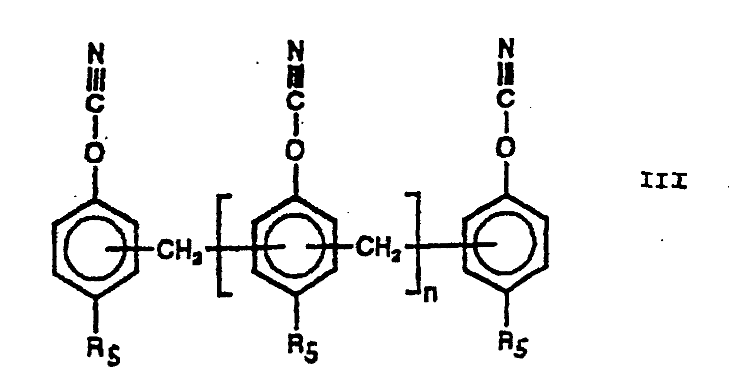 wherein R⁵ is hydrogen or C₁-C₁₀ alkyl and n is 0 to 20 and
a dicyanate of a perhalogenated dihydroxyalkane IV, in particular having up to 10 carbon atoms,
and mixtures of the polycyanates I to IV.

3. Optical element according to claim 1 or 2, **characterised in that** the polycyanurate resin is derived from a bisphenol-A dicyanate.

4. Optical elements according to one of claims 1 to 3, **characterised in that** the polycyanurate resins are co-reaction products of polycyanates with phenols, aromatic glycide ethers or glycidyl anilines.

5. Optical elements according to claim 4, **characterised in that** the polycyanurate resins, based on polycyanate used, contain 1 to 60 mole % of phenol, 1 to 99 mole % of glycide ether or 1 to 99 glycidyl aniline or mixtures of these components.

6. Optical elements according to one of the above claims, **characterised in that** the polycyanurate has a glass-transition temperature of 100 to 250°C, preferably 180 to 250°C.

7. Optical elements according to one of the above claims, **characterised by** a refractive index at 633 nm in the range from 1.45 to 1.70, preferably 1.55 to 1.65.

8. Optical elements according to one of the above claims, **characterised by** optical attenuation at 1.3 or 1.55 µm in the range from 0.1 to 1.0 dB/cm.

9. Optical elements according to one of the above claims, **characterised in that** they have been obtained by spin-coating of the dissolved polycyanurate resin.

10. Optical elements according to one of claims 1 to 8, **characterised in that** they have been obtained from the melt by moulding or embossing techniques.

11. Optical elements according to one of claims 1 to 10, **characterised in that** the polycyanurate resin has been cured thermally, optionally in the presence of a catalyst.

12. Waveguide structures, lenses to be used in the field of information technology, prisms, corrected lens systems, optical light-conducting fibres and supports for optical layers and adhesives of optical components of information technology made from a polycyanurate resin, as defined in one of claims 1 to 11.

13. Use of polycyanurate resins, as defined in one of claims 1 to 11, for producing optical elements of information technology, in particular those according to claim 11.

## Revendications

1. Eléments optiques en matière plastique pour la technique des communications, **caractérisés en ce que** la matière plastique est une résine de polycyanurate.

2. Eléments optiques suivant la revendication 1, **caractérisés en ce que** la résine de polycyanurate peut être obtenue à partir d'au moins un polycyanate ayant les formules I à IV suivantes 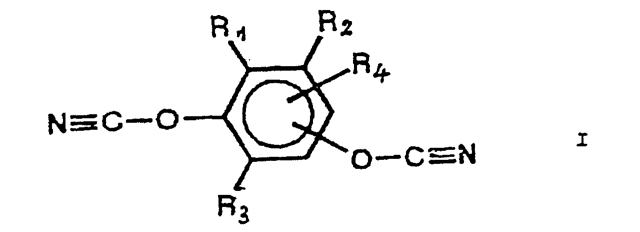 dans laquelle R¹ à R⁴ sont indépendamment l'un de l'autre l'hydrogène, alcoyle ayant de 1 à 10 atomes de carbone, cycloalcoyle ayant de 3 à 8 atomes de carbone, alcoxy ayant de 1 à 10 atomes de carbone, halogène ou phényle, les groupes alcoyle ou aryle pouvant être fluorés ou fluorés en partie, 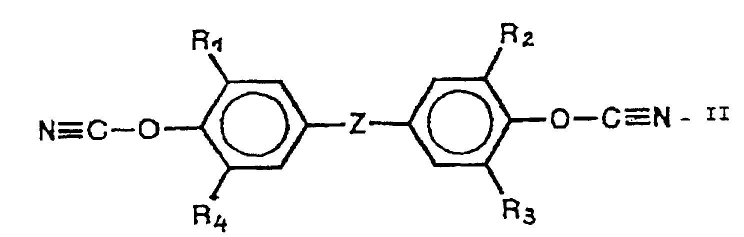 dans laquelle Z est une liaison chimique, SO₂, CF₂, CH₂, CH(CH₃), isopropyle, hexafluoroisopropyle, alcoyle ayant de 1 à 10 atomes de carbone, O, NR⁵, N=N, CH=CH, COO, CH=N, CH=N-N=CH, alcoxyalcoyle dont l'alcoyle a de 1 à 8 atomes de carbone, S ou 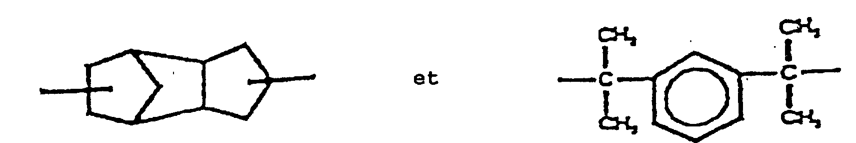 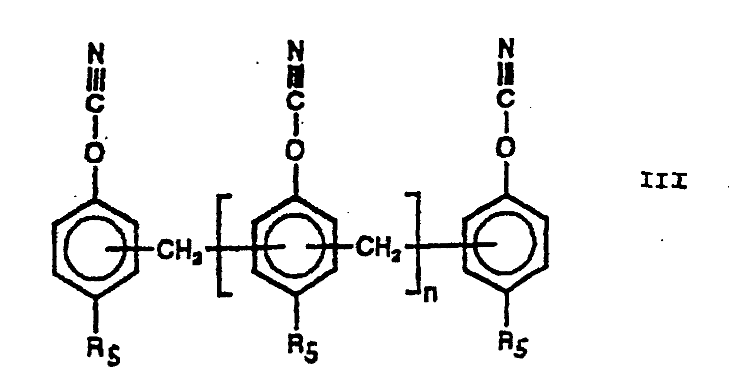 dans laquelle R⁵ est l'hydrogène ou alcoyle ayant de 1 à 10 atomes de carbone et n va de 0 à 20, ainsi que
un dicyanate d'un dihydroxyalcane perhalogéné IV, ayant notamment jusqu'à 10 atomes de carbone,
ainsi que des mélanges des polycyanates I à IV.

3. Eléments optiques suivant la revendication 1 ou 2, **caractérisés en ce que** la résine de polycyanurate dérive d'un dicyanate de bisphénol-A.

4. Eléments optiques suivant l'une des revendications 1 à 3, **caractérisés en ce que** les résines de polycyanurate sont des produits de coréaction des polycyanates sur des phénols, des oxydes glycidyliques aromatiques où des glycidylanilines.

5. Eléments optiques suivant la revendication 4, **caractérisés en ce que** les résines de polycyanurate contiennent, rapporté au polycyanate engagé, de 1 à 60 % en moles de phénol, de 1 à 99 % en moles d'oxyde glycidylique ou de 1 à 99 % en moles de glycidylaniline, ou des mélanges de ces constituants.

6. Eléments optiques suivant l'une des revendications précédentes, **caractérisés en ce que** la résine de polycyanurate a une température de transition à l'état vitreux de 100 à 250°C, de préférence de 180 à 250°C.

7. Eléments optiques suivant l'une des revendications précédentes, **caractérisés par** un indice de réfraction à 633 nm de l'ordre de 1,45 à 1,70 et, de préférence, de 1,55 à 1,65.

8. Eléments optiques suivant l'une des revendications précédentes, **caractérisés par** une atténuation optique à 1,3 ou 1,55 µm de l'ordre de 0,1 à 1,0 dB/cm.

9. Eléments optiques suivant l'une des revendications précédentes, **caractérisés en ce qu'**ils ont été obtenus par revêtement par centrifugation de la résine de polycyanurate dissoute.

10. Eléments optiques suivant l'une des revendications 1 à 8, **caractérisés en ce qu'**ils ont été obtenus par des techniques de moulage ou d'impression à l'état fondu.

11. Eléments optiques suivant l'une des revendications 1 à 10, **caractérisés en ce que** la résine de polycyanurate a été durcie thermiquement, le cas échéant en présence d'un catalyseur.

12. Structures de guide d'onde, lentilles à utiliser dans le domaine de la technique des communications, prismes, systèmes de lentille corrigée, fibres optiques et supports de couches optiques, ainsi que colles de composants optiques de la technique des communications en une résine de polycyanurate telle qu'elle est définie dans l'une des revendications 1 à 11.

13. Utilisation de résines de polycyanurate, telles qu'elles sont définies dans l'une des revendications 1 à 11, pour fabriquer des éléments optiques de la technique des communications, notamment ceux suivant la revendication 11.
